(19) Europäisches Patentamt
European Patent Office
Office européen des brevets

(11) **EP 4 537 760 A1**

(12) **EUROPEAN PATENT APPLICATION**

(43) Date of publication:
16.04.2025 Bulletin 2025/16

(21) Application number: 23203464.5

(22) Date of filing: 13.10.2023

(51) International Patent Classification (IPC):
**A61B 6/08** (2006.01) **A61B 6/00** (2024.01)
**A61B 6/58** (2024.01)

(52) Cooperative Patent Classification (CPC):
**A61B 6/08; A61B 6/4476; A61B 6/589**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Koninklijke Philips N.V.**
**5656 AG Eindhoven (NL)**

(72) Inventors:
• **LUNDT, Bernd**
**Eindhoven (NL)**
• **KOEPNICK, Johannes**
**Eindhoven (NL)**
• **MAY, Jan Marek**
**5656AG Eindhoven (NL)**

(74) Representative: **Philips Intellectual Property & Standards**
**High Tech Campus 52**
**5656 AG Eindhoven (NL)**

(54) **PATIENT POSITION DETERMINATION APPARATUS FOR A MEDICAL IMAGING SYSTEM**

(57) There is provided a patient position determination apparatus for a medical imaging system (200). The patient position determination apparatus comprises: an adjustable mount for an image capture device (110); at least one actuator configured to move the adjustable mount; and a controller configured to locate, based on configuration data relating to a setup of the medical imaging system, an intersection point at which an imaging beam (108) of the medical imaging system intersects a surface in a region to be imaged, and to control the at least one actuator to move the adjustable mount to position a field of view (112) of the image capture device relative to the intersection point. In this way, the apparatus enables proper synchronization between the positioning image captured by the image capture device and the medical image captured by the medical imaging system.

Fig. 2A

## Description

### FIELD OF THE INVENTION

The invention relates to a patient position determination apparatus for a medical imaging system, to a medical imaging system comprising the patient position determination apparatus, and to a method for determining patient position during medical imaging using the said apparatus.

### BACKGROUND OF THE INVENTION

In some medical imaging systems, a 3D camera is mounted in or near the collimator of an X-ray tube to obtain patient position information to support positioning or to detect motion or breathing of the patient. Nonetheless, opportunities exist to provide improved information on patient position to the radiographer, leading to better patient positioning and consequently to fewer disruptions to the workflow.

### SUMMARY OF THE INVENTION

There is provided, in a first aspect of invention, a patient position determination apparatus for a medical imaging system. The patient position determination apparatus comprises: an adjustable mount for an image capture device; at least one actuator configured to move the adjustable mount; and a controller configured to locate, based on configuration data relating to a setup of the medical imaging system, an intersection point at which an imaging beam of the medical imaging system intersects a surface in a region to be imaged, and to control the at least one actuator to move the adjustable mount to position a field of view of the image capture device relative to the intersection point.

In this way, the apparatus enables proper synchronization between the positioning image captured by the image capture device and the medical image captured by the medical imaging system, in particular by improving the centricity of the positioning image at multiple source-to-image distances, thereby providing improved information on patient position to the radiographer with consequent rationalization of the workflow.

The controller is further configured to control the at least one actuator to move the adjustable mount so that the field of view satisfies a predetermined relationship with the intersection point. In an example, the predetermined relationship is satisfied when a center of the field of view coincides with the intersection point, for example by appearing at the same location (within a predetermined tolerance, for example a user-dependent tolerance set according to the preferences of a particular user or typical user). The intersection point may correspond for example to the center of the X-ray image to acquire, to the center of a detector of the medical imaging system, to the center of a collimated area, or to the center of the object to be examined (e.g., anatomy). In an example, the intersection point is a point at which the imaging beam intersects a source-facing surface of the detector of the medical imaging system, and the controller is configured to locate the intersection point based on the configuration data comprising one or more of: i) a position of a focal spot of the medical imaging system; ii) a direction of the imaging beam; iii) a position of a center of the detector of the medical imaging system. In another example, the intersection point is a point at which the imaging beam intersects an object to be examined, and the controller is configured to locate the intersection point based further on the configuration data comprising depth information acquired by the image capture device (comprising for example a 3D camera).

The configuration data relating to the imaging setup may be known in advance or may be obtained using a sensor arrangement. Configuration data describing fixed geometry parameters of the medical imaging system may be known in advance. Configuration data describing variable geometry parameters of the medical imaging system may be obtained via sensors (e.g. linear position sensors and angulation sensors). Configuration data describing depth information may be acquired by the image capture device comprising for example a 3D camera. Using this information, the controller is configured to determine the direction of the central beam, the position of the focal spot, and the position of the center of the detector center. Based on the position of the center of the image capture device relative to the position of the focal spot, the controller is configured to calculate at least one tilt angle based on which the controller controls the at least one actuator to move the adjustable mount.

In an example, the at least one actuator is configured to tilt the adjustable mount around a single axis. In another example, the at least one actuator is configured to tilt the adjustable mount around multiple axes, for example two perpendicular axes. In other examples, the at least one actuator may be further configured to effect linear translation of the adjustable mount.

It will be understood that the patient position determination apparatus may be manufactured and sold as comprising the image capture device or that the image capture device may be provided as an aftermarket product to be installed into the patient position determination apparatus. The image capture device may comprise a sensor configured to acquire 3D data of the object to be examined, for example depth information that is usable to calculate the intersection point in the presence of the object. In an example, the image capture device comprises a 3D camera. In any case, the image capture device may be configured to obtain patient position information to support patient positioning or to detect motion or breathing of the patient. To that end, the patient position determination apparatus may be mountable in or near a collimator of an X-ray tube, for example.

According to a second aspect, there is provided a medical imaging system comprising the patient position determination apparatus of the first aspect. There may also be provided an X-ray apparatus or tube, or a collimator therefor, comprising the patient position determination apparatus of the first aspect.

According to a third aspect, there is provided a computer-implemented method for determining patient position during medical imaging using the apparatus of the first aspect. The method comprises: locating, based on the configuration data relating to the setup of the medical imaging system, the intersection point at which the imaging beam of the medical imaging system intersects the surface in the region to be imaged; controlling the at least one actuator to move the adjustable mount to position the field of view of the image capture device relative to the intersection point; and controlling the so-positioned image capture device to acquire an image of a patient to be examined for use in patient positioning.

According to a fourth aspect, there is provided a computing system configured to perform the method of the third aspect.

According to a fifth aspect, there is provided a computer program (product) comprising instructions which, when executed by a computing system, enable or cause the computing system to perform the method of the third aspect.

According to a sixth aspect, there is provided a computer-readable (storage) medium comprising instructions which, when executed by a computing system, enable or cause the computing system to perform the method of the third aspect. The computer-readable medium may be transitory or non-transitory, volatile or non-volatile.

By "patient positioning" is meant the act of placing the patient into a particular position or pose, whereas "patient position determination" refers to the act of determining the position or pose of the patient, or at least part of the patient, such as a particular anatomy to be examined, for example for the purposes of patient positioning.

Correspondingly, the term "object to be examined" as used herein refers to the patient or part of the patient.

The "imaging beam" may comprise any X-ray beam capable of generating an X-ray image but in one example comprises the central beam emitted from the X-ray tube.

The "medical imaging system" as described herein may be a radiographic imaging system such as a (digital) X-ray system (e.g., DXR), a (digital) fluoroscopy system, or any other X-ray system which provides sufficient information on its geometry parameters to implement the control of the adjustable mount, as described herein. In other examples, the medical imaging system may utilize multiple imaging modalities.

The term "obtaining", as used herein, may comprise, for example, receiving from another system, device, or process; receiving via an interaction with a user; loading or retrieving from storage or memory; measuring or capturing using sensors or other data acquisition devices.

The term "determining", as used herein, encompasses a wide variety of actions, and may comprise, for example, calculating, computing, processing, deriving, investigating, looking up (e.g., looking up in a table, a database or another data structure), ascertaining, and the like. Also, "determining" may comprise receiving (e.g., receiving information), accessing (e.g., accessing data in a memory), and the like. Also, "determining" may comprise resolving, selecting, choosing, establishing and the like.

The indefinite article "a" or "an" does not exclude a plurality. In addition, the articles "a" and "an" as used herein should generally be construed to mean "one or more" unless specified otherwise or clear from the context to be directed to a singular form.

Unless specified otherwise, or clear from the context, the phrases "one or more of A, B and C", "at least one of A, B, and C", and "A, B and/or C" as used herein are intended to mean all possible permutations of one or more of the listed items. That is, the phrase "A and/or B" means (A), (B), or (A and B), while the phrase "A, B, and/or C" means (A), (B), (C), (A and B), (A and C), (B and C), or (A, B, and C).

The term "comprising" does not exclude other elements or steps. Furthermore, the terms "comprising", "including", "having" and the like may be used interchangeably herein.

The invention may include one or more aspects, examples or features in isolation or combination whether specifically disclosed in that combination or in isolation. Any optional feature or sub-aspect of one of the above aspects applies as appropriate to any of the other aspects.

The above-described aspects will become apparent from, and elucidated with, reference to the detailed description provided hereinafter.

BRIEF DESCRIPTION OF THE DRAWINGS

A detailed description will now be given, by way of example only, with reference to the accompanying drawings, in which:

Figs. 1A-C illustrate use of a fixed camera for patient position determination in a medical imaging system;
Figs. 2A-C illustrate use of a tiltable camera for patient position determination in a medical imaging system;
Figs. 3A-C illustrate use of the tiltable camera with the object to be examined present;
Fig. 4 illustrates a patient position determination apparatus;
Fig. 5 illustrates calculation of a tilt angle for the tiltable camera; and
Fig. 6 illustrates a computing system that can be used in accordance with the systems and methods disclosed herein.

DETAILED DESCRIPTION OF EMBODIMENTS

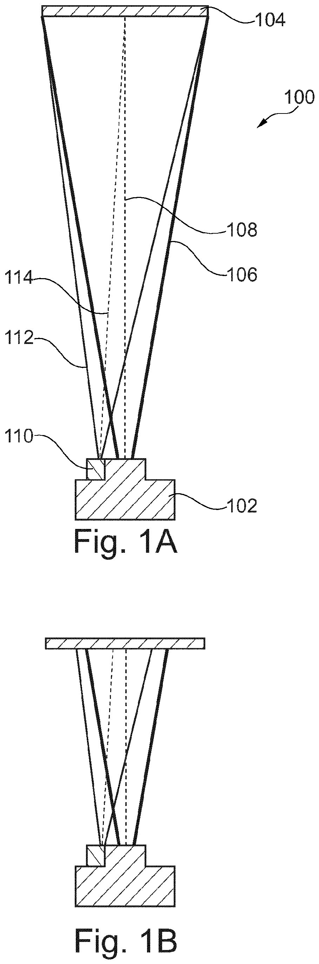

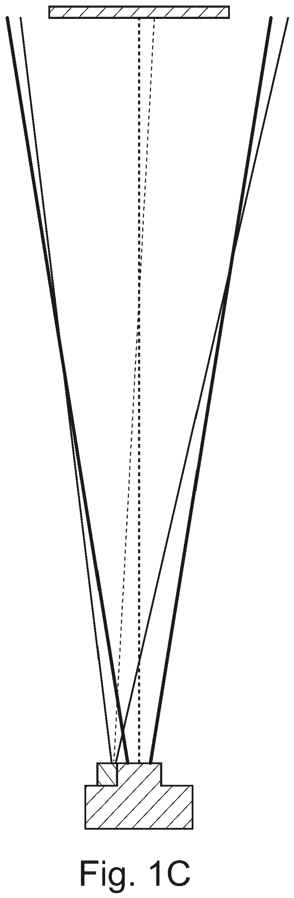

Figs. 1A-C illustrate a medical imaging system 100 comprising an X-ray tube 102 with collimator positioned facing a detector 104. The tube 102 has a region of X-ray (ROX) 106 comprising a central imaging beam or center 108. A fixed camera 110 mounted within the collimator is positioned to view the detector 104 for providing images for use in patient positioning. The fixed camera 110 has a field of view (FOV) 112 with a center of both the FOV 112 and the resulting image being indicated at 114. Due to the offset between the focal spot of the X-ray tube 102 and the lens of the fixed camera 110, the FOV center 114 coincides with the ROX center 108 only for one source-to-image distance (SID), which is the situation illustrated in Fig. 1A. For smaller or larger SIDs, as illustrated in Figs. 1B and 1C, respectively, the FOV center 114 deviates from the ROX center 108, which can be misleading for the radiographer, who expects the FOV center 114 to be at or near the ROX center 108 during visual quality checks. For small SIDs, it is furthermore possible that the anatomy to be imaged does not reside completely within the FOV 112 of the camera 110.

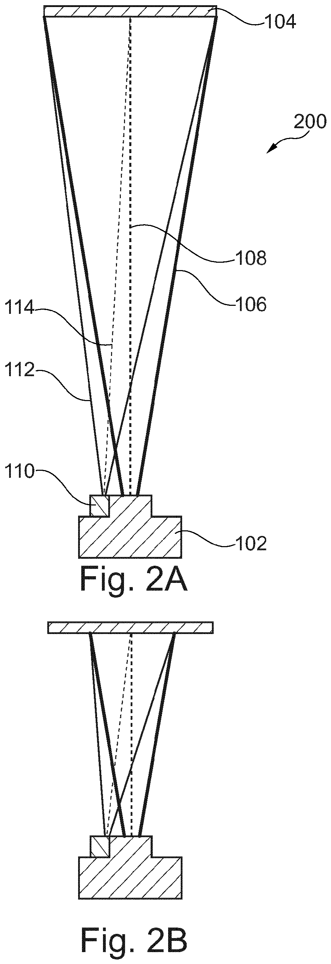

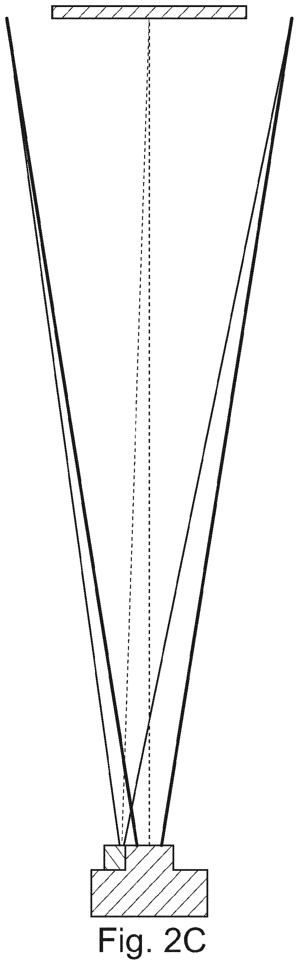

Figs. 2A-C illustrate a medical imaging system 200 according to the present disclosure. Using a tiltable mount for the camera 110, the camera 110 is tilted in such a way that the FOV center 114 coincides with the ROX center 108 at each of multiple SIDs. In particular, Figs. 2B and 2C show how tilting to the right for small SIDs and to the left for large SIDs ensures that the ROX center 108 (i.e., the point at which the central X-ray beam 108 intersects the source-facing surface of the detector 104) is shown at the FOV center 114.

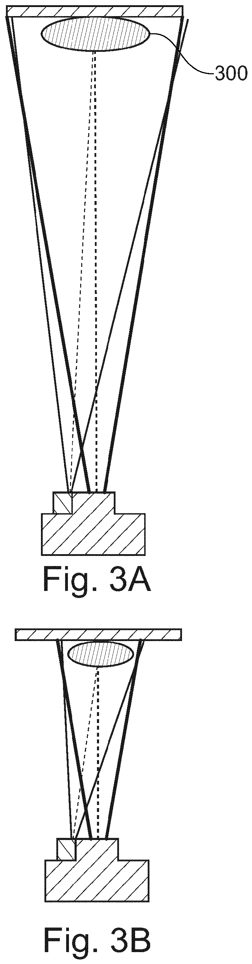

Figs. 3A-C illustrate a medical imaging system 200 of Figs. 2A-C in the case that the object to be examined 300 is present. As shown, the camera 110 is tilted using the motorized tiltable mounting such that the FOV center 114 coincides with the ROX center 108 (i.e., the point at which the central X-ray beam 108 intersects the source-facing surface of the object to be examined 300) at each of multiple SIDs.

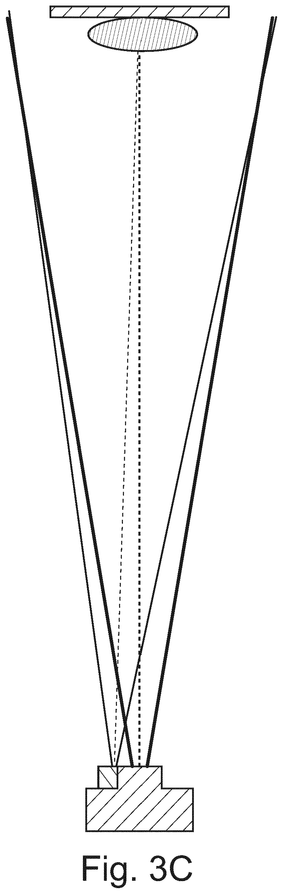

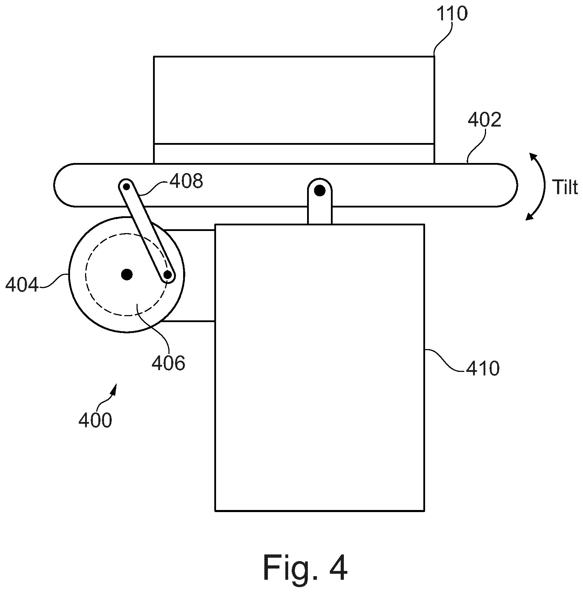

Fig. 4 illustrates one non-limiting example of a patient position determination apparatus 400. The patient position determination apparatus 400 comprises an adjustable (i.e., tiltable) mount 402 for the camera 110 and an actuator 404 configured to move the adjustable mount 402. The actuator 404 comprises a rotary servomotor 406 coupled to the tiltable mount 402 via a crank mechanism 408. A controller 410 is provided to control the actuator 404 so as to tilt the camera 110 in the manner described with reference to Figs. 2 and 3. In particular, the controller 410 is configured to obtain configuration data relating to a setup of the medical imaging system 200 and to locate, based thereon, the point at which the central imaging beam 108 intersects a source-facing surface of either the detector 104 or the object to be examined 300.

The controller 410 then controls actuator 404 to move the tiltable mount 402 to position the FOV 112 of the camera 110 relative to the located intersection point. In one non-limiting example, the controller 410 obtains from the medical imaging system 200 configuration data from which the position of the focal spot of the X-ray tube 102, the direction of the central X-ray beam 108, and the position of the detector 104 can be derived. The position of the camera sensor center relative to the position of the focal spot of the X-ray tube 102 is furthermore known to the controller 410. Using this data, the controller 410 calculates a tilt angle for the tiltable mount 402 which places the intersection point at the FOV center 114. If the object to be examined 300 is present (which is mostly the case), depth information obtained from the camera 110 is used by the controller 410 to calculate the intersection point at which the central X-ray beam 108 hits the object 300. In this case, the controller 410 calculates a tilt angle which places this intersection point at the FOV center 114.

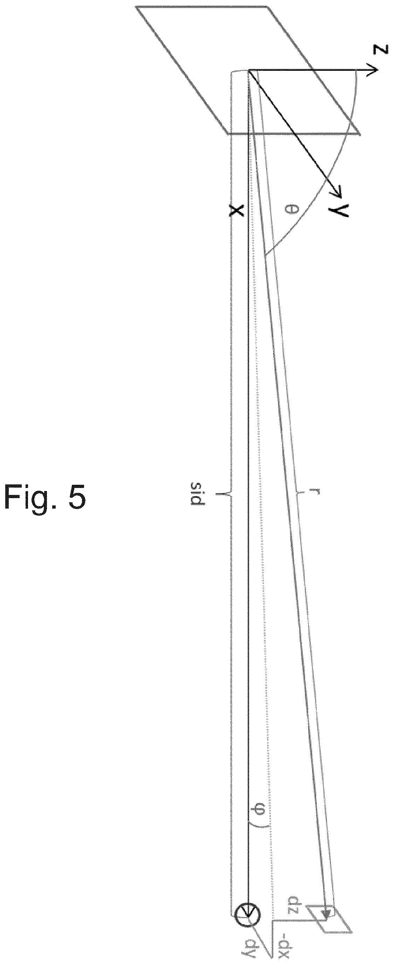

Fig. 5 illustrates calculation of the tilt angle for the tiltable camera 110 by the controller 410. In the non-limiting example of the geometry, the origin of the cartesian coordinate system is positioned at the center of the X-ray detector 104. The detector 104 lies within the y-z plane of the coordinate system. The focal spot 116 of the X-ray tube 102 is positioned at the coordinate (SID, 0, 0) and the central beam 108 of the tube 102 travels from the focal spot 116 to the center of the detector 104. The center of sensor 118 has a fixed offset (-dx, dy, dz) relative to the focal spot 116. This means that the center of the camera sensor 118 is at the position (SID - dx, dy, dz). Switching to spherical coordinates, the position of the center of the camera sensor is as follows:

$$r = \sqrt{(SID - dx)^2 + dy^2 +}$$

$$\theta = \cos^{-1} \frac{dz}{r}$$

$$\varphi = \operatorname{sgn}(dy) \cos^{-1} \frac{x}{\sqrt{(SID - dx)^2 + dy^2}}$$

To compensate the two angles $\theta$ and $\varphi$, the camera 110 is tilted by 90° - $\theta$ downwards and $\varphi$ to the left when looking from the focal spot 116 to the detector 104. In this case, the center of the detector 104 is mapped onto the center of the camera sensor 118. It will be appreciated that this non-limiting example of tilt angle calculation can be extended to cover more complex situations in which the tube 102 is angulated and/or in which the central beam 108 does not hit the center of the detector 104. It will be appreciated that variants to the arrangements described herein are conceivable. In an example, the servomotor as described herein may be replaced or supplemented by an arrangement using a piezo element or a piezo stack as active component.

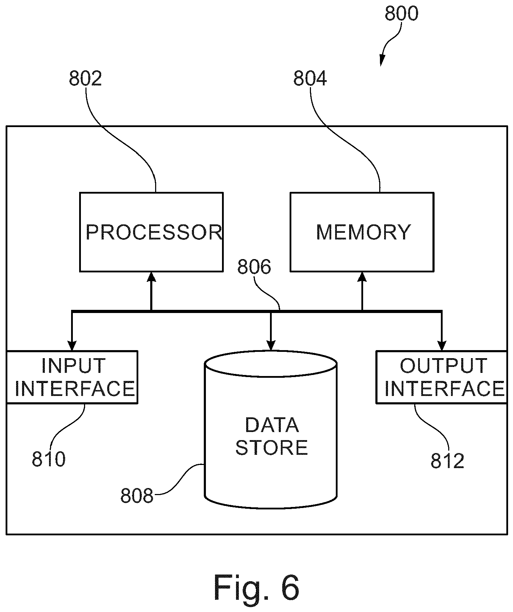

Fig. 6 illustrates an exemplary computing system 800 that can be used to implement the controller 410 as described herein. The computing system 800 may form part of or comprise any desktop, laptop, server, or cloud-based computing system. The computing system 800 includes at least one processor 802 that executes instructions that are stored in a memory 804. The instructions may be, for instance, instructions for implementing functionality described as being carried out by the controller 410 or instructions for implementing one or more of the methods described herein. The processor 802 may access the memory 804 by way of a system bus 806. In addition to storing executable instructions, the memory 804 may also store conversational inputs, scores assigned to the conversational inputs, etc.

The computing system 800 additionally includes a data store 808 that is accessible by the processor 802 by way of the system bus 806. The data store 808 may include executable instructions, log data, etc. The computing system 800 also includes an input interface 810 that allows external devices to communicate with the computing system 800. For instance, the input interface 810 may be used to receive instructions from an external computer device, from a user, etc. The computing system 800 also includes an output interface 812 that interfaces the computing system 800 with one or more external devices. For example, the computing system 800 may display text, images, etc. by way of the output interface 812.

It is contemplated that the external devices that communicate with the computing system 800 via the input interface 810 and the output interface 812 can be included in an environment that provides substantially any type of user interface with which a user can interact. Examples of user interface types include graphical user interfaces, natural user interfaces, and so forth. For instance, a graphical user interface may accept input from a user employing input device(s) such as a keyboard, mouse, remote control, or the like and provide output on an output device such as a display. Further, a natural user interface may enable a user to interact with the computing system 800 in a manner free from constraints imposed by input device such as keyboards, mice, remote controls, and the like. Rather, a natural user interface can rely on speech recognition, touch and stylus recognition, gesture recognition both on screen and adjacent to the screen, air gestures, head and eye tracking, voice and speech, vision, touch, gestures, machine intelligence, and so forth.

Additionally, while illustrated as a single system, it is to be understood that the computing system 800 may be a distributed system. Thus, for instance, several devices may be in communication by way of a network connection and may collectively perform tasks described as being performed by the computing system 800.

Various functions described herein can be implemented in hardware, software, or any combination thereof. If implemented in software, the functions can be stored on or transmitted over as one or more instructions or code on a computer-readable medium. Computer-readable media include computer-readable storage media. Computer-readable storage media can be any available storage media that can be accessed by a computer. By way of example, and not limitation, such computer-readable storage media can comprise FLASH storage media, RAM, ROM, EEPROM, CD-ROM or other optical disc storage, magnetic disk storage or other magnetic storage devices, or any other medium that can be used to carry or store desired program code in the form of instructions or data structures and that can be accessed by a computer. Disk and disc, as used herein, include compact disc (CD), laser disc, optical disc, digital versatile disc (DVD), floppy disk, and Blu-ray disc (BD), where disks usually reproduce data magnetically and discs usually reproduce data optically with lasers. Further, a propagated signal may be included within the scope of computer-readable storage media. Computer-readable media also includes communication media including any medium that facilitates transfer of a computer program from one place to another. A connection, for instance, can be a communication medium. For example, if the software is transmitted from a website, server, or other remote source using a coaxial cable, fiber optic cable, twisted pair, digital subscriber line (DSL), or wireless technologies such as infrared, radio, and microwave, then the coaxial cable, fiber optic cable, twisted pair, DSL, or wireless technologies such as infrared, radio and microwave are included in the definition of communication medium. Combinations of the above should also be included within the scope of computer-readable media.

Alternatively, or in addition, the functionally described herein can be performed, at least in part, by one or more hardware logic components. For example, and without limitation, illustrative types of hardware logic components that can be used include Field-programmable Gate Arrays (FPGAs), Application-specific Integrated Circuits (ASICs), Application-specific Standard Products (ASSPs), System-on-a-chip systems (SOCs), Complex Programmable Logic Devices (CPLDs), etc.

The applicant hereby discloses in isolation each individual feature described herein and any combination of two or more such features, to the extent that such features or combinations are capable of being carried out based on the present specification as a whole in the light of the common general knowledge of a person skilled in the art, irrespective of whether such features or combinations of features solve any problems disclosed herein, and without limitation to the scope of the claims. The applicant indicates that aspects of the present invention may consist of any such individual feature or combination of features.

It has to be noted that embodiments of the invention are described with reference to different categories. In particular, some examples are described with reference to methods whereas others are described with reference to apparatus. However, a person skilled in the art will gather from the description that, unless otherwise notified, in addition to any combination of features belonging to one category, also any combination between features relating to different category is considered to be disclosed by this application. However, all features can be combined to provide synergetic effects that are more than the simple summation of the features.

While the invention has been illustrated and described in detail in the drawings and foregoing description, such illustration and description are to be considered exemplary and not restrictive. The invention is not limited to the disclosed embodiments. Other variations to the disclosed embodiments can be understood and effected by those skilled in the art, from a study of the drawings, the disclosure, and the appended claims.

The mere fact that certain measures are recited in mutually different dependent claims does not indicate that a combination of these measures cannot be used advantageously.

Any reference signs in the claims should not be construed as limiting the scope.

**Claims**

1. A patient position determination apparatus (400) for a medical imaging system (200), the patient position determination apparatus comprising:

   an adjustable mount (402) for an image capture device (110);
   at least one actuator (404) configured to move the adjustable mount; and
   a controller (410) configured to locate, based on configuration data relating to a setup of the medical imaging system, an intersection point at which an imaging beam (108) of the medical imaging system intersects a surface in a region to be imaged, and to control the at least one actuator to move the adjustable mount to position a field of view (112) of the image capture device relative to the intersection point.

2. The patient position determination apparatus of claim 1, wherein the controller is further configured to control the at least one actuator to move the adjustable mount so that the field of view satisfies a predetermined relationship with the intersection point.

3. The patient position determination apparatus of claim 2, wherein the predetermined relationship is satisfied when a center (114) of the field of view coincides with the intersection point.

4. The patient position determination apparatus of any preceding claim, wherein the intersection point is a point at which the imaging beam intersects a source-facing surface of a detector (104) of the medical imaging system, and wherein the controller is configured to locate the intersection point based on the configuration data comprising one or more of: i) a position of a focal spot of the medical imaging system; ii) a direction of the imaging beam; iii) a position of a center of a detector of the medical imaging system.

5. The patient position determination apparatus of any preceding claim, wherein the intersection point is a point at which the imaging beam intersects an object (300) to be examined, and wherein the controller is configured to locate the intersection point based on the configuration data comprising depth information acquired by the image capture device.

6. The patient position determination apparatus of any preceding claim, further comprising the image capture device.

7. The patient position determination apparatus of claim 6, wherein the image capture device comprises a 3D camera.

8. A medical imaging system (200) comprising the patient position determination apparatus (400) of any preceding claim.

9. A computer-implemented method for patient position determination during medical imaging using the apparatus (400) of any of claims 1-7, the method comprising:

   locating, based on the configuration data relating to the setup of the medical imaging system (200), the intersection point at which the imaging beam (108) of the medical imaging system intersects the surface in the region to be imaged;
   controlling the at least one actuator (404) to move the adjustable mount (402) to position the field of view (112) of the image capture device (110) relative to the intersection point; and
   controlling the so-positioned image capture device to acquire an image of an object to be examined for use in patient positioning.

10. A computer-readable medium (804, 808) comprising instructions which, when executed by a computing system (800), cause the computing system to perform the method of claim 9.

Fig. 1A

Fig. 1B

Fig. 1C

Fig. 2A

Fig. 2B

Fig. 2C

300

Fig. 3A

Fig. 3B

Fig. 3C

Fig. 4

Fig. 5

Fig. 6

Europäisches
Patentamt

European
Patent Office

Office européen
des brevets

# EUROPEAN SEARCH REPORT

Application Number

EP 23 20 3464

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2008/122936 A1 (LOMNES STEPHEN JOHNSON [US] ET AL) 29 May 2008 (2008-05-29) * paragraph [0020] * | 1-10 | INV. A61B6/08 A61B6/00 A61B6/58 |
| A | US 5 159 622 A (SAKANIWA HIROSHI [JP] ET AL) 27 October 1992 (1992-10-27) * column 4, line 66 - column 5, line 11 * | 1-10 | |
| A | US 2019/328465 A1 (LI YAN M [US] ET AL) 31 October 2019 (2019-10-31) * the whole document * | 1-10 | |

TECHNICAL FIELDS
SEARCHED     (IPC)

A61B

The present search report has been drawn up for all claims

| Place of search | Date of completion of the search | Examiner |
|---|---|---|
| Munich | 7 March 2024 | Anscombe, Marcel |

EPO FORM 1503 03.82 (P04C01)

## ANNEX TO THE EUROPEAN SEARCH REPORT
## ON EUROPEAN PATENT APPLICATION NO.

EP 23 20 3464

This annex lists the patent family members relating to the patent documents cited in the above-mentioned European search report.
The members are as contained in the European Patent Office EDP file on
The European Patent Office is in no way liable for these particulars which are merely given for the purpose of information.

07-03-2024

| Patent document cited in search report | | Publication date | Patent family member(s) | | Publication date |
|---|---|---|---|---|---|
| US 2008122936 | A1 | 29-05-2008 | NONE | | |
| US 5159622 | A | 27-10-1992 | JP | H0683708 B2 | 26-10-1994 |
| | | | JP | H03158139 A | 08-07-1991 |
| | | | US | 5159622 A | 27-10-1992 |
| US 2019328465 | A1 | 31-10-2019 | US | 2019328465 A1 | 31-10-2019 |
| | | | WO | 2019213103 A1 | 07-11-2019 |

EPO FORM P0459

For more details about this annex : see Official Journal of the European Patent Office, No. 12/82